# EUROPEAN PATENT APPLICATION

(11) **EP 4 628 089 A1**
(43) Date of publication of application: **08.10.2025**
(21) Application number: 22967100.3
(22) Date of filing: 29.11.2022
(51) Int. Cl.: A61K 35/747, A23L 33/135, A61P 3/00, A61P 27/02

(54) **COMPOSITION FOR IMPROVING FATIGUE, SLEEPING DISORDERS OR EYE STRAIN AND MENOPAUSAL SYMPTOMS**

(71) Applicant: House Wellness Foods Corporation, Hyogo 664-0011 (JP); House Foods Group Inc., Higashiosaka-shi, Osaka 577-8520 (JP)
(72) Inventor: OHARA Tatsuya, Itami-shi, Hyogo 664-0011 (JP); NAKAJIMA Tsubasa, Itami-shi, Hyogo 664-0011 (JP); KITAMURA Kohei, Higashiosaka-shi, Osaka 577-8520 (JP); KAWASAKI Kengo, Itami-shi, Hyogo 664-0011 (JP); HIROSE Yoshitaka, Itami-shi, Hyogo 664-0011 (JP)
(74) Representative: Kraus & Lederer PartGmbB
(86) International application number: PCT/JP2022/043911
(87) International publication number: WO 2024/116265

(57) **Abstract**

A problem to be solved by the present invention is to provide a composition for improving fatigue, sleep disorders or eye strain, and menopausal symptoms. The problem is solved by a composition comprising *Lactobacillus plantarum* L-137.

## Description

### TECHNICAL FIELD

The present invention relates to a composition for improving fatigue, sleep disorders or eye strain, and menopausal symptoms.

### BACKGROUND ART

Menopausal symptoms are symptoms that appear during menopause due to a decrease in ovarian and hormone function by aging. The symptoms include, for example, vasomotor disorder-like symptoms, such as hot flashes; pains, such as headaches and myalgia; a decrease in mental health and the quality of sleep; general fatigue; and others. Menopausal symptoms occur mostly in women but are also observed in men. Regardless of menopause, many people of today are also suffering from fatigue, sleep disorders, eye strain, and other symptoms that are caused by psychological or physical stress. These symptoms are known to be improved by an extract from germinated and fermented beans (Patent literature 1).

However, the lactic acid bacterial strain *Lactobacillus plantarum* L-137 is not at all known to treat, prevent or improve fatigue, sleep disorders or eye strain, and menopausal symptoms.

### CITATION LIST

### PATENT LITERATURE

Patent literature 1: JP 2016-526019 A

### SUMMARY OF INVENTION

### TECHNICAL PROBLEM

### SOLUTION TO PROBLEM

The inventors investigated a wide variety of different materials to find out a material that has the effect of treating, preventing or improving fatigue, sleep disorders or eye strain, and menopausal symptoms. As a result, the inventors found that the lactic acid bacterial strain *Lactobacillus plantarum* L-137 (hereinafter also referred to as the "L-137 strain") surprisingly has such a desired effect. This is a surprising finding by the inventors. The inventors conducted further studies and completed the invention.

Thus, the present invention relates to the following.
[1] A composition for treating, preventing or improving one or more symptoms selected from the group consisting of fatigue, sleep disorders, and eye strain, the composition comprising *Lactobacillus plantarum* L-137 or a processed product thereof.
[2] A composition for treating, preventing or improving a menopausal symptom, the composition comprising *Lactobacillus plantarum* L-137 or a processed product thereof.
[3] The composition according to the above [2], wherein the composition is used for treating, preventing or improving one or more symptoms selected from the group consisting of the following (i) to (xii):
   (i) vasomotor disorder-like symptoms; (ii) paresthesia-like symptoms; (iii) insomnia; (iv) nervousness; (v) melancholia; (vi) vertigo/dizziness; (vii) general fatigue; (viii) arthralgia and/or myalgia; (ix) headaches; (x) palpitation; (xi) formication; and (xii) eye strain.
[4] The composition according to any one of the above [1] to [3], wherein the composition is a food or drink.
[5] The composition according to the above [4], wherein the food or drink is a food additive or a dietary supplement.
[6] Use of *Lactobacillus plantarum* L-137 or a processed product thereof for production of a medicament for treating, preventing or improving at least one symptom selected from the group consisting of fatigue, sleep disorders, and eye strain.
[7] Use of *Lactobacillus plantarum* L-137 or a processed product thereof for production of a medicament for treating, preventing or improving a menopausal symptom.
[8] A method for treating, preventing or improving at least one symptom selected from the group consisting of fatigue, sleep disorders, and eye strain by administering *Lactobacillus plantarum* L-137 or a processed product thereof to a subject.
[9] A method for treating, preventing or improving a menopausal symptom by administering *Lactobacillus plantarum* L-137 or a processed product thereof to a subject.

### ADVANTAGEOUS EFFECTS OF INVENTION

The present disclosure provides a composition for treating, preventing or improving fatigue, sleep disorders or eye strain, and menopausal symptoms. The composition of the present invention is useful for the treatment, prevention or improvement of various menopausal symptoms, such as vasomotor disorder-like symptoms, paresthesia-like symptoms, insomnia, nervousness, melancholia, vertigo/dizziness, general fatigue, arthralgia and/or myalgia, headaches, palpitation, formication, and eye strain. The present disclosure also provides a production method of the composition.

### DESCRIPTION OF EMBODIMENTS

### Composition for treating, preventing or improving fatigue, sleep disorders and/or eye strain

Preferably, the present invention is used to treat, prevent or improve fatigue, sleep disorders, and/or eye strain. Tiredness generally refers to a state of a reduction in activity or efficiency caused by excessive physical and mental burdens due to physical and/or mental causes. Fatigue is a self-sensation of such tiredness. Physical fatigue may include, for example, but is not limited to, hard exercise and muscle training, physical tiredness due to work, etc. Mental fatigue may include, for example, but is not limited to, human relationships, concerns about work, environmental changes due to moving, a job transfer, etc., and stress as a result of a life change due to marriage, childbirth, etc.

Sleep disorders may be, for example, but are not limited to, insomnia, sleep-onset insomnia, non-restorative sleep, wake after sleep onset, early awakening, nightmare, sleepwalking, hypnolepsy, sleep behavior disorder, hypersomnia, narcolepsy, respiratory-related sleep disorder, apnea, and circadian rhythm sleep disorder. Preferably, insomnia, sleep-onset insomnia, and non-restorative sleep are treated, prevented or improved by the present invention, and more preferably insomnia is treated, prevented or improved by the present invention, but the present invention is not limited thereto.

Eye strain refers to, for example, asthenopia, objectively assessed eye tiredness or subjectively assessed eye tiredness, dryness of eyes, or itching of eyes, but is not limited thereto. Eye strain is preferably one induced by light stimulus (so-called blue light stimulus) from office automation (OA) equipment such as smartphones and personal computers, but is not limited thereto. Eye strain that is preferably treated, prevented or improved by the present invention is objectively assessed eye tiredness or subjectively assessed eye tiredness, and asthenopia, but is not limited thereto.

A subject that is to receive the composition of the present invention is not limited to individuals or a population in menopause or in pre- or post-menopause, but can include all possible individuals or populations in all age categories.

### Composition for treating, preventing or improving menopausal symptoms

Preferably, the present invention is used to treat, prevent or improve menopausal symptoms. The term "menopause" as used herein generally refers to about five year-period before and after the cessation of menstruation by a reduction in ovarian activity (e.g., amenorrhea of 12 months or longer) in cases of women, but the symptoms may also be observed in women who have undergone the removal of the ovary. In the broadest sense, the term refers to a period in which the secretion level of the female sex hormone estrogen rapidly decreases, but such a period may vary with individuals or subjects within a population (e.g., depending on the race, the era, etc.) and thus is not limited to such a period. In cases of men, unlike women, they never experience the cessation of menstruation, and male sex hormones typically do not tend to rapidly decrease, but the secretion level of the male sex hormone testosterone may decrease with aging under environmental changes or stress. The term thus also refers to such a period in which men experience physical and mental illness similar to that observed in menopause in women, but such a period may vary with individuals or populations and thus is not limited to such a period.

The menopausal symptoms that are preferably treated, prevented or improved by the present invention are, for example, but not limited to, one or more selected from the group consisting of the following (i) to (xii):
(i) vasomotor disorder-like symptoms (for example, including, but not limited to, hot flashes (a sudden flare of intense heat, flushed skin, sweating, etc.), sensitivity to the cold, tachycardia, bradycardia, etc.), (ii) Paresthesia-like symptoms (for example, including, but not limited to, tingling, hyperesthesia, anesthesia, etc., and specifically including a sensation of tingling and numbness in the arms, hands or lower extremities (groin area, thighs, knees, calves, ankles, feet, etc.)), (iii) insomnia, (iv) nervousness, (v) melancholia, (vi) vertigo/dizziness, (vii) general fatigue, (viii) arthralgia and/or myalgia (for example, including, but not limited to, pain in one or more of the head, shoulder, back, waist, buttocks, lower extremities, arms, hands, etc.), (ix) headaches (for example, including, but not limited to, tension headaches, migraines, etc.), (x) palpitation, (xi) formication, and (xii) eye strain.

Among these symptoms, more preferred symptoms that are treated, prevented or improved by the present invention are insomnia and/or general fatigue, but are not limited thereto.

The present invention is preferably used to treat, prevent or improve menopausal symptoms in women and men, and is more preferably used to treat, prevent or improve menopausal symptoms in women.

### Lactobacillus plantarum strain L-137

The composition of the present invention comprises the lactic acid bacterial strain *Lactobacillus plantarum* L-137 (Accession number: FERM BP-08607) or a processed product thereof.

The lactic acid bacterial strain *Lactobacillus plantarum* L-137 (*Lactobacillus plantarum* L-137) used in the present invention was deposited with the International Patent Organism Depositary of the Incorporated Administrative Agency National Institute of Advanced Industrial Science and Technology (currently known as the International Patent Organism Depositary of the Incorporated Administrative Agency National Institute of Technology and Evaluation; address: #120, 2-5-8 Kazusakamatari, Kisarazu-shi, Chiba 292-0818, Japan) under Accession No. FERM BP-08607 (transferred from FERM P-15317 deposited on November 30, *1995). Lactobacillus plantarum* L-137 herein includes mutants of *Lactobacillus plantarum* L-137 that have the same characteristics as those of *Lactobacillus plantarum* L-137. The composition of the present invention may further contain other lactic acid bacteria in addition to *Lactobacillus plantarum* strain L-137.

The composition of the present invention preferably contains about 0.0001 to 10% by weight of *Lactobacillus plantarum* strain L-137 or a processed product thereof relative to the total amount of the composition, and more preferably contains about 0.001 to 8% by weight, or further preferably about 0.002 to 4% by weight, of the L-137 strain or a processed product thereof relative to the total amount of the composition, but the amount of the L-137 strain or a processed product thereof is not limited to these ranges.

The intake of *Lactobacillus plantarum* strain L-137 or a processed product thereof according to the present invention, when administered orally or via an injection, can be determined depending on the age and body weight of a person who will take the L-137 strain or a processed product thereof, the symptoms, the administration period, the dosage form, the mode of administration, a medicine to be co-administered, etc. For example, the intake of *Lactobacillus plantarum* strain L-137 based on the weight of dried killed bacterial cells is preferably about 0.5 to 200 mg per adult human (about 60 kg) per day, more preferably about 1 to 100 mg per adult human (about 60 kg) per day, and further preferably about 2 to 50 mg per adult human (about 60 kg) per day, but the intake is not limited thereto. The intake of *Lactobacillus plantarum* L-137 based on the number of viable bacterial cells is preferably about 5 × 10⁸ to 2 × 10¹¹ cfu (colony forming unit) per adult human (about 60 kg) per day, and more preferably about 1 × 10⁹ to 1 × 10¹¹ cfu per adult human (about 60 kg) per day, but the intake is not limited thereto. The frequency of intake may be once a day or multiple times a day. The daily dose may be administered or applied as a single dose per day or as multiple divided doses per day.

### Culture of lactic acid bacteria

The *Lactobacillus plantarum* strain L-137 and other lactic acid bacteria in the present invention may be those cultured in a culture medium such as a natural medium, a synthetic medium or a semi-synthetic medium. The culture of the lactic acid bacteria herein may be performed by a known method, a method known per se or an equivalent method thereof.

The culture medium can be any culture medium, and is preferably, for example, a culture medium containing a nitrogen source and/or a carbon source. The nitrogen source may be, for example, but is not limited to, meat extract, peptone, gluten, casein, yeast extract, amino acids, etc. The carbon source may be, for example, but is not limited to, glucose, xylose, fructose, inositol, maltose, starch syrup, yeast extract, starch, bagasse, wheat bran, molasses, glycerol, etc. These can be used alone or in combination of two or more types. The culture medium can further contain a mineral in addition to the nitrogen source and/or the carbon source. The mineral may be, for example, but is not limited to, ammonium sulfate, potassium phosphate, magnesium chloride, sodium chloride, iron, manganese, molybdenum, various types of vitamins, etc. These can be used alone or in combination of two or more types.

The culture temperature and time of the *Lactobacillus plantarum* strain L-137 and other lactic acid bacteria may be any temperature and time that allow the bacteria to be efficiently cultured. In an embodiment of the present invention, the culture temperature may be, for example, typically about 25 to 40°C, preferably about 27 to 35°C, and the culture time may be, for example, about 12 to 48 hours. In an embodiment of the present invention, the lactic acid bacteria may be cultured with aeration and shaking. The pH of the culture medium is not particularly limited, and in an embodiment of the present invention, the pH may typically be about 3 to 6, preferably about 4 to 6.

### Processed product of lactic acid bacteria

The "processed product" of the lactic acid bacterial strain *Lactobacillus plantarum* L-137 is preferably a product produced by processing the L-137 strain, including, but not limited to, a culture medium or a culture supernatant of the L-137 strain, the residue obtained after filtration or centrifugation of the culture medium or the culture supernatant, a liquid containing bacterial cells disrupted by ultrasonication, etc. The processed product according to the present invention also includes, but is not limited to, a processed liquid obtained after removing the cell walls with enzymatic or mechanical processing; protein or peptide complexes obtained after processing the bacterial cells with a chemical or by salting-out; a concentrate, a dried product, or a dilution thereof; etc.

The L-137 strain may also be viable bacterial cells, dried bacterial cells, centrifuged bacterial cells, disrupted bacterial cells, etc. and may also be killed bacterial cells, but the L-137 strain is preferably killed bacterial cells due to its stability, ease of handling, and other advantages.

The processed product as described above may directly be used, or may be formed into a powder by lyophilization, low-temperature drying, spray drying, L-drying, or a combination thereof, according to the present invention. The processed product may be diluted in an appropriate solvent (water, an alcohol, an organic solvent, etc.), or may be formed into a gel or a solid preparation by the addition of an appropriate additive.

A method for preparing killed bacterial cells of *Lactobacillus plantarum* strain L-137 and other lactic acid bacteria will be specifically described below.

The preparation method for the killed bacterial cells may be any method as long as the effects of the present invention are not lost. The killed bacterial cells may be prepared by, for example, (I) a method involving separating viable cells of the lactic acid bacteria from a liquid medium at the end of culture, and subjecting the viable bacterial cells to bactericidal treatment or sterilization treatment to kill the bacterial cells, or (II) a method involving subjecting viable cells of the lactic acid bacteria in a liquid medium to bactericidal treatment to kill the bacterial cells, and separating the killed bacterial cells from the liquid medium. Sterilization can be performed by, for example, filtration through a filter, and may be performed by other known methods, for example, gas sterilization using ethylene oxide or hydrogen peroxide; or heat sterilization using gamma rays, electron beams, high-frequency waves, or the like.

The separation method of the bacterial cells from a liquid medium may be done by various methods usually employed in this field and is not limited to a particular one. Specifically, in an embodiment of the present invention, the bacterial cells may be separated from a liquid medium by, for example, removing the supernatant by centrifugation etc. In this embodiment, if desired, after distilled water is added to the liquid medium and centrifugation is performed for the removal of the supernatant, distilled water may be added to the residue obtained by the removal of the supernatant and the resulting suspension may be further centrifuged, and this procedure may be repeated several times. In an embodiment of the present invention, the separation procedure may include filtration. The bacterial cells can be subjected to drying in a spray dryer to prepare dried bacterial cells. The spray dryer is preferably, but not limited to, a spray dryer equipped with an atomizer capable of forming spray droplets with a size of, for example, about 1 to 10 µm.

The bactericidal treatment method is not particularly limited and may be performed by any processing treatment such as heating, UV irradiation, or formalin treatment. The bactericidal treatment may be performed on harvested viable bacterial cells or on a liquid medium containing viable bacterial cells.

When the heating treatment is performed, the heating temperature may be, for example, but is not limited to, typically about 60 to 100°C, preferably about 70 to 90°C. The heating means may be those known in the art, and may be, for example, but is not limited to, a heater etc. The heating time may be any length of time that allows bactericidal treatment to be sufficiently complete, and heating may be performed, for example, typically for about 5 to 40 minutes, preferably for about 10 to 30 minutes, after the temperature reaches a desired level.

The killed bacterial cells prepared as above may be subjected to grinding, disruption, spray drying, low-temperature drying or lyophilization, or mixing with other ingredients (e.g., vitamins, amino acids, oligopeptides, etc.), or other treatments to prepare processed killed bacterial cells. In the present invention, such processed killed bacterial cells are also suitable as killed bacterial cells.

In a preferred example, the composition of the present invention is used for a food or drink and/or a medicament (including a veterinary medicine). In another preferred example, the composition of the present invention is used as an additive for a food or drink. The composition used for a food or drink, an additive for a food or drink, or a medicament can be prepared into a formulation etc. by combining the culture supernatant or a processed product thereof, or the L-137 strain or a processed product thereof according to the present invention as described above together with a pharmaceutically acceptable carrier, additive, etc., as appropriate. The formulation methods and formulation technology for preparing such a formulation are well established in the art, and the preparation of the formulation may be performed by such methods and technology. Specifically, for example, the composition used for a medicament can be formulated into an oral formulation, such as a tablet, a coated tablet, a pill, a powder, granules, a capsule, a solution, a suspension or an emulsion; or a parenteral formulation, such as an injection, an infusion, a suppository, an ointment or a patch. The blending ratio of the carrier or additive is determined as appropriate based on the amount of the carrier or additive usually used in the field of foods and drinks, medicaments or veterinary medicines.

Such pharmaceutically acceptable carrier or additive is not limited to a particular one, and examples of the carrier include various types of carriers, such as aqueous or oily bases. Examples of aqueous carriers include, but are not limited to, for example, water, physiological saline, ethanol, glycerol, polyethylene glycol, propylene glycol, methyl cellulose, hydroxypropyl methylcellulose, hydroxypropyl cellulose, polyvinylpyrrolidone, polyacrylic acid, polysaccharide gum-based natural polymers, etc. Examples of oily carriers include, but are not limited to, for example, appropriate oils and waxes, such as petrolatum, squalane, paraffin, etc.

Examples of additives include enzymes, pH adjusting agents, preservatives, bactericidal agents, antioxidants, antifungal agents, shelf life improvers, bleaching agents, brightening agents, fragrances, sweeteners, acidulants, seasonings, bittering agents, emulsifiers, thickeners, stabilizers, gelling agents, thickening agents, excipients, binders, disintegrants, lubricants, colorants, and flavor improvers. Techniques relating to such carriers or additives are well established in the art, and the carriers or additives can be used in accordance with such techniques.

When the composition of the present invention is for a food or drink, the food or drink includes health foods, foods with functional claims, foods for specified health use, and foods for sick people. The form of the food or drink is not limited to a particular one, and specific examples thereof include tablets, granules, powders, energy drinks, etc. that are ingested as so-called nutritional supplements or dietary supplements. Other examples thereof include, but are not limited to, drinks such as tea drinks, refreshing drinks, carbonated drinks, nutritional drinks, fruit juices, and lactic drinks; noodles such as buckwheat noodles, wheat noodles, Chinese noodles, and instant noodles; sweets and bakery products such as drops, candies, gum, chocolate, snacks, biscuits, jellies, jams, creams, pastries, and bread; fishery and livestock products, such as hams, sausages, hanpen fish cakes, and chikuwa fish cakes; dairy products such as processed milk and fermented milk; fats, oils and processed foods thereof, such as vegetable oils, oils for deep frying, margarine, mayonnaise, shortening, whipped cream, and dressings; seasonings such as sauces and dipping sauces; retort pouch foods such as curry, stew, rice-bowl cuisine, porridge, and rice soup; and frozen desserts, such as ice creams, sherbets, and shaved ice; etc. Techniques relating to such foods and drinks are well established in the art, and the foods and drinks can be produced and used in accordance with such techniques.

The composition of the present invention may further contain an optional ingredient known in the field of, for example, medicine, pharmaceuticals, veterinary medicine, animal science, foods, or the like to the extent that the effects of the present invention are not lost.

### Effect of improving fatigue, sleep disorders or eye strain, and menopausal symptoms and method for determining the effect

Whether the composition of the present invention has the effects according to the present invention can be determined by, for example, confirming that the composition containing *Lactobacillus plantarum* strain L-137 or a processed product thereof has better performance in improvement of fatigue, sleep disorders or eye strain, and menopausal symptoms than a composition that does not contain the L-137 strain or a processed product thereof.

Specifically, such a method for determining whether the composition has the effect of improving fatigue, sleep disorders or eye strain, and menopausal symptoms includes, but is not limited to, for example, VAS (Visual Analogue Scale) to measure subjectively assessed fatigue severity, oxidative stress marker in the blood, measurement of the time it takes a person to fall asleep (sleep latency) and the total duration of time spent sleeping (total sleep time), forced swim test (FST) using rats or mice, an evaluation method for the effect based on the questionnaire as described later and on the Kupperman's index, etc.

The effects of the composition of the present invention are not limited to treatment, prevention or improvement of menopausal symptoms, but the effects of the composition on menopausal symptoms may be evaluated by using, for example, ovariectomized animals (preferably, rats, mice, etc.) known as a menopause model. The effects of the composition on eye strain can be evaluated by, for example, a method involving irradiating blue light to retinal pigment epithelial cells or other cells of a human or an animal (e.g., rabbits etc.), and then measuring the cell viability.

For example, the composition of the present invention can be determined to have the desired effects when a measurement value of VAS or an oxidative stress marker in the blood is significantly or considerably lower or the sleep latency and/or the total sleep time is significantly or considerably longer in a subject that is allowed to ingest (or receive) the composition of the present invention than in a control group that does not ingest (or receive) the composition of the present invention.

Other than those described above, a well-established method in the art can be used to evaluate the effects of the composition. Examples of such a method are described in the Examples described later.

The composition of the present invention prepared in the form of a food or drink, a medicament (including a medicament for animals) or a quasi-drug, or its package insert or its package box, or the like may be permitted to bear claims that the food or drink, the medicament or the quasi-drug has the effect of improving fatigue, sleep disorders or eye strain, and menopausal symptoms attributed to the effects of the composition of the present invention.

### Production method of composition

The present invention includes a method for producing the composition for treating, preventing or improving fatigue, sleep disorders or eye strain, and menopausal symptoms, the method preferably comprising mixing *Lactobacillus plantarum* L-137 or a processed product thereof with a carrier and/or an excipient.

Conventional carriers are well established in the fields of foods and medicine, and the present invention may also use such conventional carriers, and a preferred carrier used in the mixing step may be various types of carriers, such as aqueous or oily bases. Examples of aqueous carriers include, but are not limited to, for example, water, physiological saline, ethanol, glycerol, polyethylene glycol, propylene glycol, methyl cellulose, hydroxypropyl methylcellulose, hydroxypropyl cellulose, polyvinylpyrrolidone, polyacrylic acid, polysaccharide gum-based natural polymers, etc. Examples of oily carriers include, but are not limited to, for example, appropriate oils and waxes, such as petrolatum, squalane, paraffin, etc.

Conventional excipients are well established in the fields of foods and medicine, and the present invention may also use such conventional excipients, and a preferred excipient used in the mixing step may be, but is not limited to, lactose, sucrose, D-mannitol, corn starch, powdered cellulose, calcium hydrogen phosphate, calcium carbonate, or the like.

The composition of the present invention can be produced and processed as appropriate in the same manner as in a common production method for a composition except that *Lactobacillus plantarum* strain L-137 or a processed product thereof is contained in the composition. In other words, the present invention also includes a method for producing the composition of the present invention, the method comprising mixing *Lactobacillus plantarum* strain L-137 or a processed product thereof with, if desired, other ingredients.

The present invention includes aspects in which the configurations described above are combined in various ways to achieve the effects of the present invention without departing from the technical scope of the present invention. Various modifications are possible as appropriate within the technical scope of the present invention.

### EXAMPLES

The present invention will be described more specifically with reference to the following Examples and Test Examples, but the present invention is not limited thereto.

### Test Examples: Human clinical study using composition containing lactic acid bacterial strain L-137 (Evaluation of menopausal symptoms and eye strain)

### 1. Composition used

A commercially available tablet containing 10 mg of heat-treated killed bacterial cells of *Lactobacillus plantarum* strain L-137 per tablet (300 mg) (trade name: Mamori Takameru Lactic acid bacteria L-137 Supplement) was used as the composition of the present invention. The tablet also contained other ingredients (lactose, starch, sucrose fatty acid ester, etc.) in addition to the L-137 strain, but these additional ingredients do not significantly affect the experiments as described below.

### 2. Ingestion of composition and evaluation by using menopausal symptom questionnaire

Twenty-two men and women aged 41 to 73 years old were divided into two groups (a non-ingestion group of six participants (one man and five women), and an L-137 strain killed bacterial cell ingestion group of 16 participants (five men and 11 women)). The L-137 strain killed bacterial cell ingestion group ingested the composition described above at a dose of one tablet a day for consecutive 12 months.

Evaluation by using the Kupperman's menopausal symptom questionnaire was performed before the start of the study and 3, 6 and 12 months after the start of the ingestion of the composition. Changes in the scores in the questionnaire from the start of the study were also examined. Symptoms included in the menopausal symptom questionnaire are the following 11 items: (1) vasomotor disorder-like symptoms, (2) paresthesia-like symptoms, (3) insomnia, (4) nervousness, (5) melancholia, (6) vertigo/dizziness, (7) general fatigue, (8) arthralgia and/or myalgia, (9) headaches, (10) palpitation, and (11) formication.

The results are shown in Table 1 below.

**Table 1**

| | | 3 months | | 6 months | | 12 months | | 2-way ANOVA | | |
|---|---|---|---|---|---|---|---|---|---|---|
| | | Mean ± SD. | n | Mean ± SD. | n | Mean ± SD. | n | Test food | Ingestion period | Interaction |
| 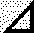 **Total score of 11 items** | Non-ingestion | 0.50 ± 4.59 | 6 | 3.33 ± 8.24 | 6 | 2.50 ± 6.19 | 6 | 0.060 | 0.470 | 0.843 |
| | Ingestion | -2.64 ± 4.55 | 14 | -1.36 ± 5.98 | 14 | 0.29 ± 7.32 | 14 | | | |
| | | | | | | | | | | |
| 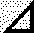 **Insomnia** | Non-ingestion | 0.17 ± 0.98 | 6 | 0.50 ± 0.84 | 6 | 0.50 ± 0.84 | 6 | 0.031 | 0.600 | 0.995 |
| | Ingestion | -0.50 ± 1.02 | 14 | -0.21 ± 0.80 | 14 | -0.14 ± 1.51 | 14 | | | |
| | | | | | | | | | | |
| 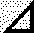 **General fatigue** | Non-ingestion | 0.00 ± 0.00 | 6 | 0.33 ± 0.52 | 6 | 0.17 ± 0.41 | 6 | 0.057 | 0.557 | 0.759 |
| | Ingestion | -0.21 ± 0.58 | 14 | -0.14 ± 0.53 | 14 | -0.07 ± 0.73 | 14 | | | |

Discussion: The indexes indicating menopausal symptoms regarding the "total score of 11 items" (i.e., the items (1) to (11) described above) and the items "insomnia" and "general fatigue" increased in the L-137 non-ingestion group over time, whereas an increase in these indexes was significantly suppressed or tended to be suppressed in the L-137 ingestion group (analyzed by 2-way ANOVA).

### 3. Ingestion of composition and evaluation by using questionnaire regarding eye strain

Twenty-two men and women aged 41 to 73 years old were divided into two groups (a non-ingestion group of six participants (one man and five women), and an L-137 strain killed bacterial cell ingestion group of 16 participants (five men and 11 women)). The L-137 strain killed bacterial cell ingestion group ingested the composition described above at a dose of one tablet a day for consecutive 12 months.

Evaluation by using a questionnaire regarding eye strain was performed before the start of the study and 3, 6 and 12 months after the start of the ingestion of the composition. Changes in the scores in the questionnaire from the start of the study were also examined. The questionnaire regarding eye strain also included the items "dryness of eyes" and "itching of eyes" in addition to "eye strain."

The results are shown in Table 2 below.

**Table 2**

| | | 3 months | | 6 months | | 12 months | | 2-way ANOVA | | |
|---|---|---|---|---|---|---|---|---|---|---|
| | | Mean ± SD. | n | Mean ± SD. | n | Mean ± SD. | n | Test food | Ingestion period | Interaction |
| 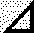 **Eye Strain** | Non-ingestion | 0.67 ± 1.21 | 6 | 0.33 ± 0.82 | 6 | 0.60 ± 1.34 | 5 | 0.033 | 0.705 | 0.642 |
| | Ingestion | -0.15 ± 0.38 | 13 | 0.00 ± 0.55 | 14 | 0.21 ± 0.89 | 14 | | | |

Discussion: The index regarding eye strain increased over time in the L-137 non-ingestion group, whereas an increase in the index was significantly suppressed in the L-137 ingestion group (analyzed by 2-way ANOVA).

The Test Examples as described above revealed that the composition containing the lactic acid bacterial strain L-137 of the present invention exhibits the effect of improving menopausal symptoms and eye strain.

### INDUSTRIAL APPLICABILITY

The composition of the present invention has various useful effects as described above. Thus the composition of the present invention is useful for the treatment, prevention or improvement of, for example, (i) vasomotor disorder-like symptoms; (ii) paresthesia-like symptoms; (iii) insomnia; (iv) nervousness; (v) melancholia; (vi) vertigo/dizziness; (vii) general fatigue; (viii) arthralgia and/or myalgia; (ix) headaches; (x) palpitation; (xi) formication; and (xii) eye strain. The composition is also useful as a food or drink, a medicament, a quasi-drug, etc.

## Claims

1. A composition for treating, preventing or improving one or more symptoms selected from the group consisting of fatigue, sleep disorders, and eye strain, the composition comprising *Lactobacillus plantarum* L-137 or a processed product thereof.

2. A composition for treating, preventing or improving a menopausal symptom, the composition comprising *Lactobacillus plantarum* L-137 or a processed product thereof.

3. The composition according to claim 2, wherein the composition is used for treating, preventing or improving one or more symptoms selected from the group consisting of the following (i) to (xii):
(i) vasomotor disorder-like symptoms; (ii) paresthesia-like symptoms; (iii) insomnia; (iv) nervousness; (v) melancholia; (vi) vertigo/dizziness; (vii) general fatigue; (viii) arthralgia and/or myalgia; (ix) headaches; (x) palpitation; (xi) formication; and (xii) eye strain.

4. The composition according to any one of claims 1 to 3, wherein the composition is a food or drink.

5. The composition according to claim 4, wherein the food or drink is a food additive or a dietary supplement.

6. Use of *Lactobacillus plantarum* L-137 or a processed product thereof for production of a medicament for treating, preventing or improving at least one symptom selected from the group consisting of fatigue, sleep disorders, and eye strain.

7. Use of *Lactobacillus plantarum* L-137 or a processed product thereof for production of a medicament for treating, preventing or improving a menopausal symptom.

8. A method for treating, preventing or improving at least one symptom selected from the group consisting of fatigue, sleep disorders, and eye strain by administering *Lactobacillus plantarum* L-137 or a processed product thereof to a subject.

9. A method for treating, preventing or improving a menopausal symptom by administering *Lactobacillus plantarum* L-137 or a processed product thereof to a subject.
